# EUROPEAN PATENT SPECIFICATION

(11) **EP 4 379 061 B1**
(45) Date of publication and mention of the grant of the patent: **27.05.2026**
(21) Application number: 22849282.3
(22) Date of filing: 14.07.2022
(51) Int. Cl.: C12Q 1/04, C12Q 1/06, C12Q 1/22, C12Q 1/689, C12M 1/34

(54) **METHOD AND APPARATUS FOR RAPIDLY TESTING MICROORGANISM**
VERFAHREN UND VORRICHTUNG ZUM SCHNELLEN TESTEN VON MIKROORGANISMEN
PROCÉDÉ ET APPAREIL POUR TESTER RAPIDEMENT UN MICRO-ORGANISME

(30) Priority: 28.07.2021 JP 2021123126
(43) Date of publication of application: 05.06.2024
(73) Proprietor: HITACHI HIGH-TECH CORPORATION, Tokyo 105-6409 (JP)
(72) Inventor: ISHIMARU Masako, Tokyo 100-8280 (JP); KAWABE Shunsuke, Tokyo 100-8280 (JP); NODA Hideyuki, Tokyo 105-6409 (JP)
(74) Representative: Mewburn Ellis LLP
(86) International application number: PCT/JP2022/027760
(87) International publication number: WO 2023/008219

(56) References cited:
- EP-A1- 3 608 418
- EP-A1- 3 926 032
- WO-A1-2009/105063
- WO-A1-2020/166130
- WO-A1-95/27797
- JP-A- 2020 130 000
- JP-A- S53 139 785
- US-A1- 2008 241 871
- NICHOLS W W ET AL: "Analysis of the disagreement between automated bioluminescence-based and culture methods for detecting significant bacteriuria, with proposals for standardizing evaluations of bacteriuria detection methods", JOURNAL OF CLINICAL MICROBIOLOGY, vol. 15, no. 5, 1 May 1982 (1982-05-01), US, pages 802 - 809, XP093273684, ISSN: 0095-1137, DOI: 10.1128/jcm.15.5.802-809.1982

## Description

### Technical Field

This application claims priority to Japanese Patent Application No. 2021-123126 filed on July 28, 2021.

The present invention relates to a method and an apparatus for detecting a microorganism in a sample.

### Background Art

In a sterility test for confirming that bacteria or fungi (hereinafter, simply referred to as "bacteria" or "microorganisms") are not mixed in pharmaceutical products, cosmetics, foods, and the like, it is necessary to detect a very small number of bacteria. A culturing method conventionally used for a sterility test is a method of culturing bacteria in a culture medium to increase the number of cells and detecting the bacteria. With this method, there has been a major problem that culture for one day or more is required to obtain a sufficient number of bacteria for detection, and it takes one week or more to confirm the negativity, which means it takes considerable time. Therefore, in recent years, several rapid test methods have been developed.

To provide a simplified test, a method of detecting CO₂ has been known. This method detects bacteria by adding a sample to a liquid culture medium placed in a sealed container and detecting carbon dioxide produced by the bacteria during their growth (PTL 1).

In addition, a detection method by an ATP (Adenosine Triphosphate) method has been known as a method capable of detecting bacteria with high sensitivity. The ATP method is a method for detecting ATP of bacteria via bioluminescence by a luciferin-luciferase reaction, and it is generally possible to detect bacteria with about 100 CFU (Colony forming unit).

For example, PTL 2 discloses a method of filtering a sample through a filter to collect bacteria on the filter and concentrating the bacteria, further eliminating extracellular ATP to remove background ATP, and then extracting intracellular ATP. The method of PTL 2 can detect bacteria with high sensitivity, and can detect even several bacteria.

### Citation List

### Patent Literature

PTL 1: US 6,074,870 B
PTL 2: US 9,290,789 B

JP S53/139785 discloses a method for detecting bacteria in blood based on the presence of bacterial ATP in a sample.

JP 2020/130000 discloses cell detection device and cell detection method.

EP 3 608 418 discloses a method for detecting a microorganism in a test sample for containing cells.

WO 2009/105063 discloses systems, methods and apparatus for determining whether a culture in a vessel contains a plurality of microorganisms.

WO 95/27797 discloses methods for both internally and externally photostandardizing chemical assays e.g. an ATP-bioluminescence assay.

US 2008/241871 discloses a method and apparatus for measuring luminescence that can be accurately and quickly carried out while inhibiting a possible background associated with viable bacteria adhering to a nozzle or Adenosine Tri Phosphate remaining in the nozzle.

W.W. Nichols et al, "Analysis of the Disagreement between Automated Bioluminescence-Based and Culture Methods for Detecting Significant Bacteriuria, with Proposals for Standardizing Evaluations of Bacteriuria Detection Methods", (1982*)* discloses a fully automated method for detecting significant bacteriuria using firefly luciferin and luciferase to detect bacterial ATP in urine.

### Summary of Invention

### Technical Problem

In a case where bacteria are detected by quantifying a bacteria-derived marker substance, such as a metabolite, a gene, or a component of bacterial cells, if the presence of bacteria can be detected in a smaller amount, a highly sensitive and rapid test may be achieved.

However, the number of microorganisms to be detected in the sterility test is as small as 100 CFU or less, and thus the amount of the marker substance is also very small. In many cases, there is a problem that the marker substance contained in a test sample itself interferes with the detection of the bacteria-derived marker substance. For example, in a case where the amount of the marker substance contained in the test sample itself is larger by one digit than the amount of the marker substance possessed by the bacteria, it is necessary to perform an operation of culturing bacteria under enrichment culture conditions until the marker substance possessed by the bacteria is significantly increased by the enrichment culture, and then measuring the marker substance.

At this time, the threshold for bacteria detection is set based on the amount of the marker substance contained in the test sample. Ideally, the detection threshold is set based on the amount of the marker substance contained in the test sample itself and its variation. For example, it is common that the amount of a marker substance in a negative control (bacteria-free test sample) is measured a plurality of times, an average value a and a standard deviation α are calculated, and the calculation is performed by setting the lower detection limit as a + 3.3σ.

However, the present inventors have considered that a problem in the product using raw materials derived from living organisms is that the value of negative control varies day by day or lot by lot. For example, in a small amount of product such as a custom-made cell preparation, the amount that can be used for the sterility test is very small, and it is not possible to estimate the variations in the values by performing measurement a plurality of times.

Therefore, the present invention provides a technique for appropriately setting a threshold for bacteria detection and rapidly detecting bacteria.

### Solution to Problem

In order to solve the above problems, the present inventors have obtained findings that bacteria can be rapidly and appropriately detected by dividing a test sample into at least two, performing different treatments on the divided samples such that there is a difference in the amount of the marker substance between the samples when bacteria are present, measuring the amount of the marker substance, using a measurement value of one of the samples for calculation of a threshold, comparing a measured value of the other sample with the threshold, and using the resulting measured value to determine the presence or absence of bacteria.

Aspects of the invention are set out in the appended claims.

### Advantageous Effects of Invention

The method and apparatus for detecting a microorganism according to the present disclosure make it possible to rapidly detect a microorganism even in a case where only a small amount of the microorganism is present in a sample or in a case where only a small amount of the marker substance to be measured is present. Therefore, the present invention may be applicable to fields such as pharmaceutical and cosmetic production, in particular, production of cell products and production of foods.

### Brief Description of Drawings

[FIG. 1] FIG. 1 is a graph showing an example in which variations in the amount of a cell-derived marker substance (ATP) in a cell sample is measured.
[FIG. 2] FIG. 2 is an example of a flowchart of a method for detecting bacteria (microorganisms) in a cell sample.
[FIG. 3] FIG. 3 is a flowchart of a method for detecting bacteria in a cell sample based on the amount of ATP in Example 1.
[FIG. 4] FIG. 4 is a graph showing an example in which bacteria in a cell sample are detected.
[FIG. 5] FIG. 5 is a schematic view of a configuration example of a microorganism detection apparatus of Example 2.
[FIG. 6] FIG. 6 is a schematic view of a configuration example of a measurement device in the microorganism detection apparatus of Example 2.
[FIG. 7] FIG. 7 shows an example of a database stored in a storage device of the microorganism detection apparatus.
[FIG. 8] FIG. 8 shows an example of a screen display of an input/output device of the microorganism detection apparatus.

### Description of Embodiments

The present invention relates to a method and an apparatus for detecting a microorganism. The present invention is characterized by a method for determining a threshold as a reference for detection when a microorganism in a sample is detected based on the amount of a marker substance present in the microorganism or secreted by the microorganism. Hereinafter, a specific description will be given.

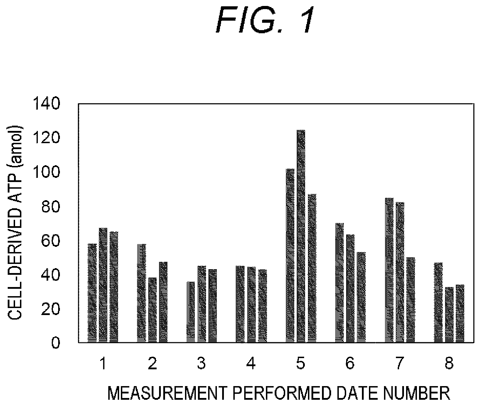

FIG. 1 shows the results of measuring the amount of ATP by performing the following operations on a bacteria-free cultured T cell suspension three times a day and eight times on different days. Specifically, to detect bacteria using ATP as a marker substance from a suspension containing 10⁶ cultured T cells, cell-derived ATP was eliminated from the cell suspension as follows. First, a cell lysis solution was added to the cell suspension to lyse the cells, and the cells were filtered through a centrifugal filter. The filter is a filtration filter having a pore size of 0.45 µm, and when bacteria are contained in the sample, the bacteria are captured on the filter. Next, filtration was conducted by adding a washing solution to the filter and lysed cells remaining on the filter were removed. Further, an ATP eliminating reagent (ATPase solution) was added to the filter, free ATP was degraded for 40 minutes, and the solution was removed by filtration. An ATP extraction reagent (reagent for disrupting bacteria and extracting intracellular ATP) was added to the resultant product for ATP extraction, and the resultant was filtered to obtain a filtrate as a measurement sample in a tube for measuring ATP. An ATP luminescent reagent was added to the resulting sample, and the amount of ATP was quantified from the amount of luminescence. The reagents and filters used in the above operations were sterilized. The T cells are produced from one cell line continuously passaged under certain culture conditions.

Since the sample used here is a bacteria-free sample, the measured amount of ATP (FIG. 1) corresponds to ATP derived from cells remaining without being removed by filtration and the ATP eliminating reagent. On the same day, measurement was performed three times for samples dispensed from one cell suspension , and the standard deviation of the diurnal variation was 9.4 amol (attomol, 10⁻¹⁸ mol). Meanwhile, the standard deviation of the interday variation was 22 amol, which was larger than the diurnal variation. It is considered that the diurnal variation is caused by variations in sampling, cell removal operation, and ATP measurement, and the interday variation reflects a variation in the state of the cells. As described above, even a sample passaged with the same cell line has a large difference in state depending on the day.

Therefore, it is ideal that the threshold for bacteria detection is a value obtained by adding the standard deviation of the diurnal variation to the average value of the ATP measured values of the sample on the day of test. For example, when ten times the standard deviation is used for calculation of the threshold, the threshold can be set to 63.4 + 10 × 4.7 = 110.4 amol based on the average value (63.4 amol) and the standard deviation (4.7 amol) of the measurement on the first day. When the threshold is calculated in the same manner as described above, the threshold can be set for each cell state on the day. For example, the threshold can be set to 145.1 amol on the second day or can be set to 90.1 amol on the third day.

However, it is actually difficult to set such a threshold. The reason for this is that the presence or absence of bacteria is unknown in the actual test sample, and it is not a measurement of pure negative control. Further, measurement is performed a plurality of times for each test sample, and the average value and variation are calculated, as a result of which the number of times of measurement increases and the cost increases. Furthermore, in a case where the amount of the target product is small and the amount of the test sample that can be used for the sterility test is small, it is difficult to perform measurement a plurality of times and calculate the variation in measurement.

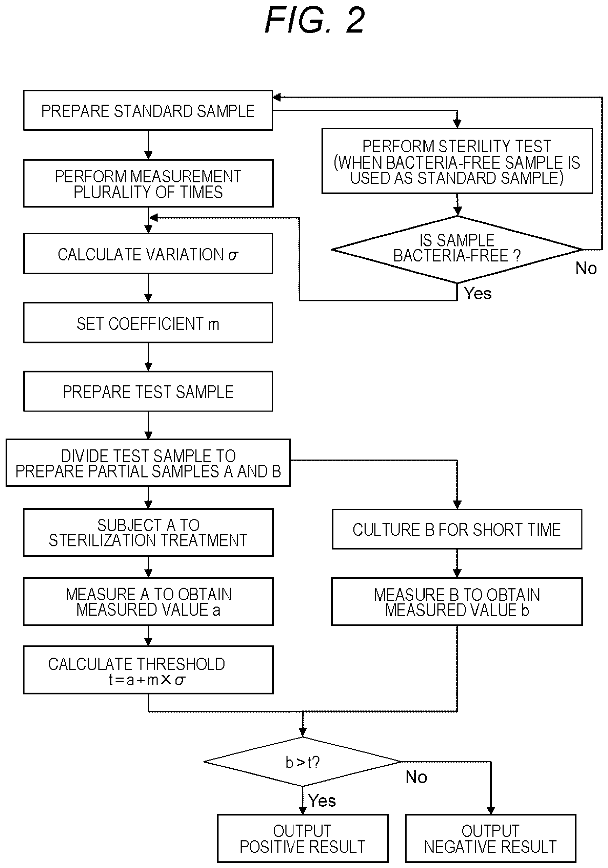

Therefore, according to the present invention, the threshold may be set by the method as shown in FIG. 2.

First, a standard sample is prepared. A sample of the same type as the test sample may be used as the standard sample. The term "same type" refers to a sample of the same type, for example, a sample containing cells of the same type. As an example, in the case of testing a cultured cell sample in biopharmaceutical production, the sample may be a sample of another lot of the same cell line as the test sample, and a plurality of lots may be prepared and used. Further, for example, in the case of testing a T cell preparation of autologous cells, T cells of the same subject may preferably be used as the same type. However, when the amount of the sample to be used for measurement is limited, established T cells, T cells provided from another subject, or the like may be used. Furthermore, for example, in the case of testing a beverage product such as fruit juice, a product produced from a raw material of the same lot, another lot, or a plurality of lots may be used.

In one embodiment, the standard sample may be a bacteria-free sample. In this case, the standard sample is separately subjected to a sterility test by a culture method or the like, and the bacteria-free state of the sample is confirmed.

The term "sterility test" as used herein refers to that in a case where culture is attempted under certain sterility test conditions, there is no bacteria that proliferate up to a detectable number. Actually, viable bacteria that do not grow under the test conditions and viable bacteria at a concentration less than or equal to the lower detection limit may be included. In this case, when the amount of marker substance possessed by these bacteria does not affect the threshold setting by the method described below and the determination of positive/negative using the threshold, the amount may be considered to be equivalent to sterility. In the case of using a bacteria-free sample or a sample equivalent to the bacteria-free sample, the test target marker substance measured contains no bacteria-derived marker substance or contains the bacteria-derived marker substance only to the extent that it does not affect the measurement, and thus it may be possible to more accurately obtain variations in the marker substance derived from the sample.

In another embodiment, the standard sample may be a non-bacteria-free sample. In the case of the non-bacteria-free sample, when the contained bacteria are uniformly dispersed and the marker substance does not increase or decrease during measurement, the sample can be used as the standard sample. In this case, when the amount of the marker substance in the standard sample is measured, the absolute value may be a value obtained by summing the value from the standard sample and the value from bacteria, but the variation may be derived from the sample, and the standard deviation σ described below can be calculated. In fact, it is assumed that a variation in dispersion of contained bacteria and a variation in marker substance occur, but when the variation resulting from the variations may be sufficiently smaller than the variation of the sample itself (e.g. 1/10 or less), the sample can be used as the standard sample. This is useful, for example, when a sample that is unlikely to be bacteria-free, such as a fermented food or a blood sample collected from a human, is a sample to be tested.

Next, for the standard sample, a marker substance (e.g. ATP) to be tested may be measured a plurality of times. More desirably, the standard sample may be measured on different days or for a plurality of lots. From the measurement result, the standard deviation σ may be calculated as a variation value (value of diurnal variation calculated excluding interday variation when measured on multiple days).

Then, a coefficient m for threshold calculation may be set. The coefficient m will be described later.

Next, a test sample may be prepared, the test sample may be divided, and at least two partial samples may be prepared. Different treatments may be applied to the at least two partial samples. When bacteria are contained, the treatments may be performed to cause a difference between the measured values of the test target marker substance of the two partial samples. In the example of FIG. 2, the amount of the bacteria-derived marker substance may be reduced by subjecting a partial sample A to a sterilization treatment, and the amount of the bacteria-derived marker substance may be increased by culturing a partial sample B for a short time. When the difference between the two measured values is larger than a determination value t calculated from a predetermined variation σ, the sample may be determined to be positive for bacteria, whereas when the difference is smaller than the determination value t, the sample may be determined to be negative for bacteria.

As described above, according to the present invention, a sample may be divided, and when a microorganism to be detected is present, different treatments may be performed on the divided samples such that there is a difference in the amount of the marker substance between the divided samples when the microorganism to be detected is present. The amount of the marker substance may be measured, a measured value of one of the samples may be used for calculating a threshold, and a measured value of the other sample may be compared with the threshold and used to determine the presence of the microorganism.

Thus, in one embodiment, the present invention provides a method for detecting a microorganism in a sample including: preparing a test sample; dividing the test sample to prepare at least two partial samples; performing different treatments on the first partial sample and the second partial sample; measuring a marker substance in the first partial sample, the marker substance being present in the microorganism or being secreted by the microorganism, to obtain a first measured value; measuring the marker substance in the second partial sample to obtain a second measured value; calculating a threshold from the first measured value; and comparing the second measured value with the threshold to determine whether or not the microorganism is contained in the sample.

In the present invention, the term "microorganism" to be detected refers to various species of microorganisms including bacteria, actinomycetes, and fungi. Specific examples of the microorganism may include microorganisms to be detected by a sterilization method in the pharmacopoeia, and microorganisms such as pathogenic bacteria and pathogenic fungi to be tested in a hospital laboratory or the like. For example, the microorganism may include bacteria and fungi such as *Pseudomonas* (as a specific example, *Pseudomonas aeruginosa), Propionibacterium* (as a specific example, *Proprionibacterium acnes), Staphylococcus* (as specific examples, *Staphylococcus aureus* and *Staphylococcus epidermidis), Micrococcus, Streptococcus* (as specific examples, *Streptococcus pyogenes* and *Streptococcus pneumoniae), Enterococcus* (as specific examples, *Enterococcus faecium* and *Enterococcus faecalis*), *Neisseria* (as specific examples, *Neisseria gonorrhoeae* and *Neisseria meningitidis), Moraxella, Escherichia* (as a specific example, *Escherichia coli), Shigella* (as a specific example, *Shigella), Salmonella* (as specific examples, *Salmonella enterica Typhi, Salmonella enterica* subsp. *Paratyphi A,* and *Salmonella enteritidis), Citrobacter, Klebsiella* (as a specific example, *Klebsiella pneumoniae), Enterobacter, Serratia* (as a specific example, *Serratia marcescens), Proteus, Providencia, Morganella, Yersinia* (as a specific example, *Yersinia pestis), Vibrio* (as specific examples, *Vibrio cholerae, Vibrio parahaemolyticus, Vibrio vulnificus,* and *Vibrio mimicus), Aeromonas, Acinetobacter* (as a specific example, *Acinetobacter baumannii), Alcaligenes, Agrobacterium, Flavobacterium, Haemophilus* (as a specific example, *Haemophilus influenzae), Pasteurella, Francisella, Bordetella* (as a specific example, *Bordetella pertussis), Eikenella, Brucella, Streptobacillus, Actinobacillus, Legionella* (as a specific example, *Legionella pneumophila), Bacillus* (as specific examples, *Bacillus subtilis, Bacillus anthracis,* and *Bacillus cereus), Corynebacterium* (as a specific example, *Corynebacterium diphtheriae), Lactobacillus, Listeria, Erysipelothrix, Nocardia, Actinomyces, Clostridium* (as specific examples, *Clostridium perfringens* and *Clostridium sporogenes), Bacteroides* (as a specific example, *Bacteroides fragilis), Fusobacterium, Mycobacterium* (as a specific example, *Mycobacterium tuberculosis), Campylobacter, Helicobacter* (as a specific example, *Helicobacter pylori), Spirillum, Treponema, Borrelia, Leptospira, Mycoplasma* (as a specific example, *Mycoplasma pneumonia), Aspergillus* (as specific examples, *Aspergillus niger* and *Aspergillus brasiliensis),* yeast (as a specific example, *Candida albicans),* but microorganisms other than these can also be the target for detection. According to the present invention, among indicator bacteria in the pharmacopoeia, a microorganism (such as *Pseudomonas aeruginosa*) with a small amount of ATP, whose ATP level is difficult to detect, can be detected by culture in a short time.

The test sample may not be particularly limited as long as the presence or absence of a microorganism in the test sample is desired to be determined. The test sample may include, in particular, a biological sample derived from an organism, a sample which is suspected of being contaminated with a microorganism, and a sample which should be determined to be negative for the presence of a microorganism. For example, the sample may be various samples such as a cell sample (e.g. stem cells, T cells, grafts, cell culture solutions, or cell culture supernatants), a body fluid sample (e.g. whole blood, serum, plasma, urine, bone marrow fluid, semen, breast milk, amniotic fluid, saliva), a pharmaceutical or cosmetic sample (e.g. injections, infusions, eye drops, raw materials, production intermediates, or container cleaning solutions), an environmental sample (e.g. clean room air or surface), a culture medium sample, a food sample (e.g. foods, beverages, feeds, supplements, or intermediates in the production process), a water sample (e.g. seawater, river water, or industrial water), and a soil sample. Further, in the case of the sample derived from an organism, the origin of the test sample may not be particularly limited, and the sample may be derived from any organism species. For example, a sample derived from at least one organism of various species such as animals, plants, and insects may be used as the test sample.

In the present invention, the term "detection" means to determine whether or not a microorganism is present in the test sample, to estimate the amount of the microorganism in the test sample, to confirm that the microorganism is not present in the test sample (negative for microorganisms), and the like. The concentration of the microorganism that may be contained in the test sample may not be particularly limited.

The term "marker substance" to be measured means a substance present in or secreted by a microorganism to be detected, and is a substance whose amount or concentration in a sample can be measured. The marker substance varies depending on the type of microorganism to be detected, the type of sample, measuring instrument, and the like, and those skilled in the art can appropriately select the marker substance. Examples thereof may include ATP, endotoxin, nicotinamide adenine dinucleotide (such as reduced NAD(P)H), genes (such as DNA, RNA, and miRNA), proteins (such as β-galactosidase, NADH-quinone oxidoreductase, other enzymes, and surface proteins expressed on the surface of microorganisms), sugar chains, membrane lipids, and metabolites. Methods and means for measuring such marker substances are known in the art.

In the present method, a test sample may be first prepared. When the test sample is a liquid sample, the sample can be used as it is, or used after being diluted or concentrated with a solvent. In the case of a solid sample, a supernatant obtained by suspending the sample in a solvent, homogenizing the sample with a pulverizer or the like, or stirring the sample and a solvent may be used. Examples of the solvent may include distilled water, physiological saline, a phosphate buffer solution, and a Tris buffer. Furthermore, it may also be possible to use a filter through which a sample is filtered and on which a microorganism is captured. Such a filter may not be particularly limited as long as it has a size capable of capturing the microorganism.

Next, the test sample may be divided to prepare at least two partial samples. For example, the test sample may be divided into two. The divided partial samples may be placed in separate containers. A case where a sample is divided into two to prepare a first partial sample and a second partial sample will be described herein in detail. However, when the amount of the test sample is large, a third partial sample, a fourth partial sample, and the like may be prepared, and the treatment can be performed similarly.

Next, different treatments may be performed on the divided partial samples. Specifically, different treatments may be performed on the first partial sample and the second partial sample. Here, the term "different treatments" means a treatment which causes a difference in the amount or concentration of the marker substance to be measured later, when the microorganism is present in each of the test samples. The operation of the treatment may vary, the treatment conditions (time, temperature, reagent to be added, etc.) may vary, the number of treatment operations may vary, or one of the samples may be treated and the other sample may be untreated. In one embodiment, a treatment for inhibiting the measurement of the marker substance may be performed on the partial sample (e.g. the first partial sample). Examples of the treatment may include, but is not limited to, sterilization treatment (e.g. heat treatment, treatment with ultraviolet rays, gamma rays or electron beam, or treatment with a bactericide), bacteriostatic treatment (e.g. treatment for suppressing the growth of viable bacteria, such as heat treatment or treatment with a bacteriostatic agent), and treatment with an antibody that inhibits proteolysis or detection (when the marker substance is a protein). In one embodiment, a treatment for increasing an amount of the marker substance may be performed on the partial sample (e.g. the second partial sample). The treatment for increasing the amount of the marker substance may preferably be performed when the amount of the marker substance present in the microorganism is small or when the variation due to the diurnal variation is large. The treatment may not be limited, but examples thereof may include culturing under enrichment conditions. The culture medium used for culture varies depending on the type of microorganism to be detected and the type of test sample. For example, a culture medium (such as a soybean-casein digest (SCD) medium, a Sabouraud glucose liquid medium, a liquid thioglycolic acid medium, or lactose broth) used in the existing sterility test method can be used. Those skilled in the art can appropriately select the culture medium depending on the type of the test sample, the type of microorganism that may be present, the assumed amount of microorganism, and the like. The culture can be carried out under conditions conventionally used in the art (temperature, time, aerobic or anaerobic culture, static or shake culture). Specific culture conditions may vary depending on the type of target microorganism, the type of test sample, the type of culture medium to be used, and the like, and those skilled in the art can set the conditions appropriately. The culture time may be specifically 30 minutes to 48 hours, preferably 1 to 30 hours, and more preferably 1 to 24 hours. The culture time varies depending on the amount and type of microorganism to be detected, the type of test sample, the culture medium to be used, the culture temperature, and the like.

The different treatments may preferably be set such that the amount of the marker substance in the first partial sample is less than the amount of the marker substance in the second partial sample. In a preferred embodiment, the first partial sample may be subjected to sterilization or bacteriostatic treatment, and the second partial sample may be cultured under enrichment conditions. In another preferred embodiment, the first partial sample may be subjected to sterilization or bacteriostatic treatment, and the second partial treatment may not be treated (untreated). In still another preferred embodiment, the culture time of the first partial sample may be set to be shorter than the culture time of the second partial sample.

In one embodiment, the treatment to be performed on the second partial sample may include a treatment for increasing an amount of the marker substance, and the present method may further include determining a time of the treatment for increasing the amount of the marker substance of the microorganism, based on the known amount of the marker substance of the microorganism, the known growth rate of the microorganism, and the variation σ. Since the doubling time of microorganisms, the amount of the marker substance, and the like vary depending on the type of microorganisms. Thus, the treatment time may be desirably determined depending on the type of microorganisms.

Subsequently, the marker substance in the first partial sample may be measured to obtain a first measured value. Further, the marker substance in the second partial sample may be measured to obtain a second measured value. These steps may be performed in parallel or continuously. Performing some or all of the steps in parallel may make it possible to perform the test in a shorter time. The treatment for changing the amount of the marker substance for each partial sample may be performed in parallel, and the measurement of the marker substance may be continuously performed. It does not matter which of the first measured value and the second measured value is obtained first. Alternatively, after obtaining the first measured value and calculating the threshold to be described later, the second measured value may be obtained.

The method for measuring the marker substance varies depending on the type of the marker substance, and those skilled in the art can appropriately measure the marker substance. For example, when the marker substance is ATP, the measurement of ATP can be performed by a known ATP luminescence measurement method. Specifically, luciferase and luciferin which chemically react with ATP to produce light may be used, luminescence produced by the reaction of ATP in the cells with luciferase, and luciferin may be measured, and the ATP value may be determined from the amount of luminescence. Those skilled in the art can easily measure such ATP using a commercially available ATP detection reagent or kit. As the pretreatment, it may be preferable to eliminate ATP other than microorganism-derived ATP, and for example, ATPase may be added to a sample. Subsequently, intracellular ATP may be extracted using a known ATP extract (such as trichloroacetic acid, a surfactant, or lysozyme). Thereafter, the extracted ATP may be made to emit light using the luciferin-luciferase luminescence reaction. Specifically, a luminescent reagent containing luciferin and luciferase may be added to the sample. The luminescent reagent may react with the extracted ATP to produce luminescence, and thus the amount of luminescence may be measured. The luminescent reagent is also known in the art and is not particularly limited. The measurement of luminescence can be performed by any luminescence measurement method and photodetector known in the art, for example, using a luminometer, a spectrophotometer, or a luminescence plate reader. The ATP value in the sample may be obtained based on the measured value of the amount of luminescence.

When the marker substance is endotoxin, measurement of the substance is well known in the art, and many detection kits and detection systems are commercially available. Preferably, a method or kit capable of measuring endotoxin by optical measurement may be used.

Even in a case where the marker substance is a gene (such as DNA, RNA, or miRNA), the measurement of the gene amount is well known in the art. For example, there are many known methods in which principles of polymerase chain reaction (PCR), hybridization, and the like are applied. Those skilled in the art can measure the gene as the marker substance by an appropriate method according to the gene to be measured. Kits containing primers for PCR amplification of the common region of bacterial or fungal 16S ribosomal DNA are commercially available, and bacterial detection by PCR may be possible. After the bacterial detection, the bacterial species can also be identified by subjecting the PCR product to sequencing.

When the marker substance is a protein (such as a surface protein, β-galactosidase, and other enzymes), the marker substance can be measured using an antigen-antibody reaction, an enzyme reaction, or the like depending on the type of the protein. Measurement of such proteins is also well known in the art, and many detection kits and detection systems are commercially available. Preferably, a method or kit capable of measuring proteins by optical measurement may be used.

When the marker substance is a metabolite, the metabolite can be measured by a means for measuring a physical or chemical characteristic specific to the metabolite, for example, a means for measuring an accurate molecular weight or an NMR spectrum. As the means for measuring the metabolite, analytical devices such as a mass spectrometer (MS), an NMR analyzer, a two-dimensional electrophoresis device, chromatograph, and a liquid chromatograph mass spectrometer (LC/MS) are known. When the metabolite is ATP, the ATP can be measured by luminescence measurement with the ATP measurement kit described above. Further, when the metabolite is a reactive chemical substance, for example, a substance such as nicotinamide adenine dinucleotide, e.g. reduced nicotinamide adenine dinucleotide (NAD (P) H), there are a method including: adding a dye that reacts with the substance and changes the fluorescence wavelength; and measuring the fluorescence, and a method including: adding a reagent that reacts with the substance to form an active oxygen; and measuring the active oxygen by chemiluminescence.

Subsequently, a threshold may be calculated from the first measured value. The threshold is important in determining the presence of microorganisms based on the difference in the amounts of marker substance in the partial samples subjected to the above-described different treatments. Thus, the threshold may be calculated in consideration of the variation caused by the variation in the amounts of the marker substance in the test samples and the variation in the measurements of the amount of the marker substance. The variation may preferably be obtained based on the variation within the measurement date. This variation can be determined in advance using a sample of the same type as the test sample or a portion of the test sample, or can be determined in parallel with the present method. When the variation caused by the variation in the amounts of the marker substance in the test sample is sufficiently smaller than the variation in the measurements of the amount of the marker substance, the threshold may be calculated using only the variation in the measurements of the amount of the marker substance.

For example, a case where the variation of the sample is obtained using a sample of the same type as the test sample, and then the threshold value is calculated will be described. In one embodiment, the method may further include: preparing, as a standard sample, a sample of the same type as the test sample; measuring the marker substance in the standard sample a plurality of times; calculating a variation σ of a plurality of resulting measured values; calculating an average value a of the first measured values; and calculating the threshold t by t = a + m · σ (where m is any coefficient).

The standard sample may be as described above, and may be, for example, a sample of another lot of the same product as the test sample, and a plurality of lots may be prepared and used. This standard sample may be a sample that has been confirmed to be bacteria-free, or may be a sample that is not bacteria-free.

Next, the marker substance in the standard sample may be measured a plurality of times. The plural measurements may preferably be performed on the same day to grasp the diurnal variation. More specific understanding on the variation can be achieved by performing the measurements on different days or performing the measurement on a plurality of lots. The variation σ (standard deviation) may be calculated based on the plural measured values obtained.

The average value of the first measured values as described above may be calculated, and the threshold t may be calculated by t = a + m · σ. Here, the coefficient m represents how many times a difference between a measured value (e.g. a first measured value) of one of the partial samples and a measured value (e.g. a second measured value) of the other partial sample is higher than the variation σ to determine that the microorganism is present in each of the samples. For example, in a case where the microorganism is determined to be present when the difference is more than or equal to ten times the variation σ, the coefficient m is 10. m may be any numerical value, and may be more than or equal to a detection limit: 3, and may be preferably 6 or more, more preferably 9 or more. The larger the value m, the lower the false positive rate. Meanwhile, the sensitivity (rate to be determined as positive for bacteria, in bacteria-positive samples) may decrease, and thus the value m may be desirably 20 or less.

Although a specific example for calculating the threshold has been described, the threshold can be calculated by another calculation method in the present method, and the calculation method is not limited to the above-described method.

Thereafter, the second measured value may be compared with the above-described threshold, thereby determining whether or not the microorganism is contained in the sample. Specifically, when the second measured value is smaller than the above-described threshold, it may be determined that no microorganism is present in the sample (negative for microorganisms). Meanwhile, when the second measured value is more than or equal to the above-described threshold, it may be determined that the microorganism is present in the sample (positive for microorganisms). Alternatively, for example, when the coefficient m is 5 to 10 and the second measured value is the same as or approximately the same as the threshold described above, remeasurement may be performed. When the second partial sample is measured a plurality of times, the average value of the second measured values may be used for the determination.

According to the present method, it may be possible to detect a microorganism in a rapid manner and/or a high-sensitivity manner, even in a case where only a small amount of the microorganism is present in a sample or in a case where only a small amount of the marker substance to be measured is present.

Furthermore, the present invention provides a microorganism detection apparatus for performing the method as described above. Specifically, the microorganism detection apparatus according to the present invention includes: a measurement unit that is configured to measure a marker substance in at least two partial samples prepared by dividing a test sample, the marker substance being present in the microorganism or being secreted by the microorganism; a storage unit that is configured to store a value of variation in marker substance measured value in a sample of the same type as the test sample, the value being obtained in advance; and a control/analysis unit, in which the control/analysis unit is configured to calculate a detection threshold based on a measured value of one of the partial samples measured by the measurement unit and the variation value from the storage unit, and to compare a measured value of the other partial sample measured by the measurement unit with the threshold, and determine whether or not the microorganism is contained in the test sample.

The measurement unit may include a means capable of measuring at least a marker substance. Such a measurement means may vary depending on the type of marker substance to be measured, and examples thereof may include a photodetector (such as a luminometer, a spectrophotometer, or a luminescence plate reader) and a mass spectrometer. The measurement unit may be configured to measure the marker substance for each of a plurality of partial samples. For example, the measurement unit may include a plurality of measurement means, or a plurality of partial samples may be measured by one measurement means.

The storage unit may be configured to store at least a value of variation in marker substance measured value for a sample of the same type as the test sample, the value being obtained in advance. The storage unit may be configured to store a measured value (actual value) of the marker substance when the stored variation value is calculated, the method and conditions of measuring the marker substance, the type of test sample, and the like for each type of marker substance. Further, the measured value of the marker substance in the test sample newly measured by the present apparatus, the measurement method and conditions, the type of the test sample, the determination result, and the like may be stored as needed.

The control/analysis unit may not be particularly limited as long as it can control the entire apparatus or control the operation in each component (such as the measurement unit or the storage unit) and/or execute analysis, and may generally be a computer. The control/analysis unit may be arranged or integrated by connection to other components (such as the measurement unit and the storage unit), or may be connected via a wired or wireless network.

The control/analysis unit may be configured to calculate a detection threshold based on a measured value (e.g. the first measured value of the first partial sample) of one of the partial samples measured by the measurement unit and the variation value from the storage unit, and to compare a measured value (e.g. the second measured value of the second partial sample) of the other partial sample measured by the measurement unit with the threshold, and determine whether or not the microorganism is contained in the test sample. A specific determination method may be similar to the microorganism detection method described above.

The apparatus may further include a sample treatment unit having at least two sample containers, in which, in the sample containers of the sample treatment unit, different treatments are performed on the at least two partial samples. For example, the sample treatment unit can include a unit for separating cells and a microorganism contained in each of the samples (such as a filter or a centrifugal separator), a container for collecting a marker substance to be measured, a temperature control device, and the like, in addition to the sample containers. Further, the sample treatment unit may include a unit (such as a dispensing pipette) for dividing the test sample.

The apparatus may further include an input unit and/or an output unit. The input unit may be used to input, to the control/analysis unit, at least one type of information selected from the group consisting of a type of the test sample, a type of the marker substance, and a method for measuring the marker substance. Further, the output unit may output, for example, a determination result in the control/analysis unit.

Note that the present apparatus may include a portion holding a reagent used in the measurement unit and a unit for introducing the reagent. For example, in the case of measuring ATP as the marker substance, it may be possible to include a portion for holding an ATP detection reagent (such as ATPase, ATP extract, or ATP luminescent reagent) and a unit for introducing the ATP detection reagent into each of the partial samples.

The present apparatus may include a mechanism for transporting a sample or a container containing a sample or a container containing a marker substance between the sample treatment unit and the measurement unit.

The present apparatus can rapidly detect a microorganism in a sample. In particular, even in a case where the amount of the microorganism contained in the sample is small or in a case where the amount of the marker substance to be measured is small, the present apparatus has the advantage of rapidly detecting the microorganism.

### Examples

Hereinafter, the present invention will be described more specifically with reference to Examples and the drawings. However, the Examples below do not limit the scope of the present invention.

### Example 1

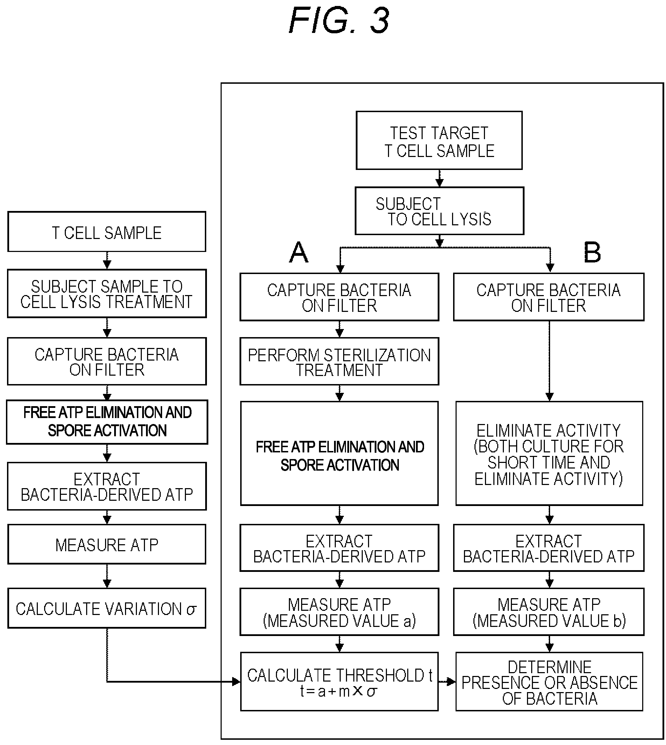

FIG. 3 shows a specific example in which bacteria in a T cell culture solution were detected using the method of the present invention. In this example, ATP was measured as a test target marker substance.

First, as a standard sample (T cell sample), a T cell culture solution containing 10⁶ T cells was measured three times a day, the amount of ATP was measured on a plurality of different days, and the standard deviation σ in the day was calculated as σ = 9.4 amol (FIG. 1). The method was the same as that described for FIG. 1, and the sample was subjected to cell lysis treatment, bacteria were captured on a filter, the activity of ATP was eliminated (ATP elimination), bacteria-derived ATP was extracted, the ATP level was measured, and the standard deviation (variation) σ was calculated (left flow in FIG. 3).

Next, a T cell culture solution containing 34 CFU of *Pseudomonas aeruginosa* (*P. aeruginosa*) and 2 × 10⁶ T cells was prepared as a test sample (T cell sample to be tested). This sample was subjected to cell lysis treatment in the same manner as in the standard sample, and then the resulting sample was equally divided, and two partial samples A and B were prepared. Then, the samples were added to separate centrifugal filters, and a washing solution was added to each of the filters. Thus, the lysed cells remaining on the filter were removed.

Next, the filter of the sample A was subjected to a heat sterilization treatment. The ATP eliminating reagent (ATPase solution) was then added to both the filters, the filters were incubated at 35°C, and free ATP was degraded over 180 minutes. Simultaneously, viable bacteria present in the sample B were cultured.

Next, the liquid was removed by filtration, and an ATP extraction reagent (reagent for disrupting bacteria and extracting intracellular ATP) was added thereto to form a bacterial ATP solution. Then, the solution was filtered to give a filtrate in a tube for measuring ATP. An ATP luminescent reagent was added to the resulting sample, and the amount of ATP was quantified from the amount of luminescence.

Bacteria in the partial sample A are disrupted by subjecting the partial sample A to the sterilization treatment. Accordingly, the measured value of the partial sample A does not include bacteria-derived ATP, and only cell-derived ATP is measured. Further, the bacteria in the partial sample B proliferate in the ATP eliminating reagent, thereby an increase in ATP. Thus, a measured value b of the partial sample B includes bacteria-derived ATP and cell-derived ATP.

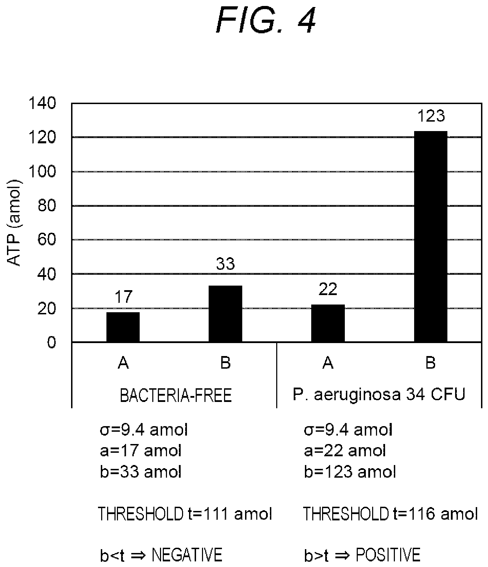

The measurement results are shown in the graph of FIG. 4. In the case of the sample containing 34 CFU of bacteria and 2 × 10⁶ T cells, a measured value a of the partial sample A was 22 amol, and the measured value b of the partial sample B was 123 amol (right side in FIG. 4). Here, the value a may be used instead of the average of the ATP content of the test sample itself, which is required upon calculating the detection threshold, and the standard deviation σ measured in advance with the standard sample may be used as the value of variation. In a case where it is set that the sample is determined to be positive for bacteria when there is a difference of more than or equal to ten times in the deviation σ between the value of the partial sample A and the value of the partial sample B (i.e. the coefficient m is set to 10), and the threshold t may be calculated by t = a + m × σ = 22 + 10 × 9.4 = 116 amol. Since the value b of the partial sample B was larger than the threshold t, the sample was determined to be positive for bacteria. Here, for example, when the threshold t satisfies t = b, the sample may be suspected to be positive for bacteria, and the sample may be retested.

In a case where the same test was performed using a test sample: T cell culture solution containing 2 × 10⁶ T cells without containing bacteria, the value a was 17 amol, and the value b was 33 amol (left side in FIG. 4). Similarly, the threshold was calculated, the obtained value was 111 amol, and the value b was smaller than the obtained value, so the sample was determined to be negative for bacteria.

Here, in the threshold calculation method: t = a + m · σ, the value of the coefficient m may be set to 3.29 or 3 as the method of calculating the detection limit. This value may be a value when the number of times of measurement at the time of calculating the deviation σ is sufficient, and it may be necessary to set m to about 6 in the case of measurement of n = 3. Further, the threshold may be calculated using the value a of one measurement of the partial sample A instead of the average value of plural measurements. Thus, the threshold may preferably have a likelihood of about 3 σ for correction. Therefore, it may be desirable that a value m is 6 or more, more preferably 9 or more. The larger the value m, the lower the false positive rate. Meanwhile, the sensitivity (rate to be determined as positive for bacteria, in bacteria-positive samples) may decrease, and thus the value m may be desirably 20 or less.

The amount of the marker substance possessed by bacteria in the partial sample B may be necessary to be higher than that of the partial sample A by m · σ or more. When the amount of the marker substance of bacteria is small or when the deviation σ due to the diurnal variation is large, the false positive rate can be reduced by increasing the value b through enrichment culture.

In this example, the partial sample A was subjected to sterilization treatment, and the partial sample B was subjected to enrichment culture. However, the partial sample B may not be necessary to be subjected to enrichment culture in a case where it is found that the amounts of bacteria-derived ATP in the partial samples A and B are largely different by σ, even if the enrichment culture is not performed, such as a case where the lower detection limit of the target bacteria is high. In this case, the elimination of the activity can be shortened to, for example, 30 minutes for each of the partial samples A and B, and thus the presence or absence of bacteria can be determined more rapidly.

In addition, the same purpose can be achieved by performing a treatment for blocking the detection of the detection target substance instead of the sterilization treatment of the partial sample A. For example, when an antigen on the surface of bacteria is detected by the antigen-antibody reaction, it may also be possible to adopt a treatment for degrading the antigen, a treatment for adding an antibody that binds to the antigen and inhibits detection by a detection reagent, and the like.

Alternatively, it may also be possible to adopt a method of providing a difference in enrichment time in which the sterilization treatment or the like is not performed on the partial sample A, and the time of the ATP elimination of the partial sample A is set to 30 minutes, the time of the ATP elimination of the partial sample B is set to 180 minutes, and the like.

The treatments of the partial samples A and B may preferably be performed in parallel. This is because viable bacteria captured on the filters may grow or produce ATP on the filters when conditions are met, and the amount of ATP may change with time. Further, the partial samples A and B may be treated in parallel, rather than sequentially, and thus the treatment time may be shortened and a rapid test can be achieved. The time difference between the start of the treatment of the partial sample A and the start of the treatment of the partial sample B may preferably be within 10 minutes. This is because it takes about 10 to 20 minutes for E. *coli* that divide rapidly among bacteria to divide, and it may become difficult to perform accurate measurement when the time difference increases.

In a case where the partial samples A and B are not treated in parallel, and some or all of the partial samples A and B are treated in series, some ingenuity may be needed so as not to affect the determination result due to an unexpected increase in viable bacteria during the treatment. For example, the treatment of the partial sample B may be started after finishing the sterilization treatment of the partial sample A, and thus it may be possible to prevent the growth of bacteria in the partial sample A and an increase in the measured value. Conversely, for example, in a case where the sample contains a substance that suppresses the growth of bacteria such as an antibiotic, the antibiotic can be removed by first filtrating and washing the partial sample B. Accordingly, the enrichment of the bacteria in the partial sample B can be achieved.

In addition, the content of ATP in the bacteria may vary depending on the bacterial species. For example, the content of ATP in *Staphylococcus* (bacteria belonging to the genus *Staphylococcus*) may be 1 to 10 amol per 1 CFU, and the content of ATP in *Candida* (fungi belonging to the genus *Candida*) may be 50 to 500 amol per 1 CFU. The doubling time of bacteria (the time at which the number of bacteria or the marker substance to be measured is doubled) may also be different for each of the bacterial species. The doubling time of *Staphylococcus* may be 10 to 30 minutes, and the doubling time of *Candida* may be 1 to 2 hours. It may be possible to set the enrichment culture time based on the information regarding these species.

First, for the bacterial species to be detected, the content of ATP, and the time of cell division may be measured and stored in a database. Next, a value of m · σ used for threshold calculation may be calculated. In the example of the T cell of this example, 10 × 9.8 = 98 amol. In the case of the target lower detection limit of 30 CFU, 15 CFU are present in the partial sample B and grow during the enrichment culture. For example, in the case of detecting *Staphylococcus aureus,* the amount of bacteria-derived ATP in the partial sample B before enrichment culture is 1 amol/CFU × 15 CFU = 15 amol, which is lower than the threshold. The 15 CFU may grow with doubling time of 30 minutes, and the amount may reach 120 amol after culture for 1.5 hours. Therefore, it can be seen that the enrichment culture time needed may be 1.5 hours. Actually, the enrichment culture time may be desirably set to about 2 hours to 3 hours with a margin, in consideration of variations in dividing the test sample of 30 CFU, enrichment culture conditions, variations among strains, and the like.

As described above, the amount of the bacteria-derived marker substance to be measured and the growth rate of bacteria are set in advance, whereby it is possible to set the enrichment culture time.

### Example 2

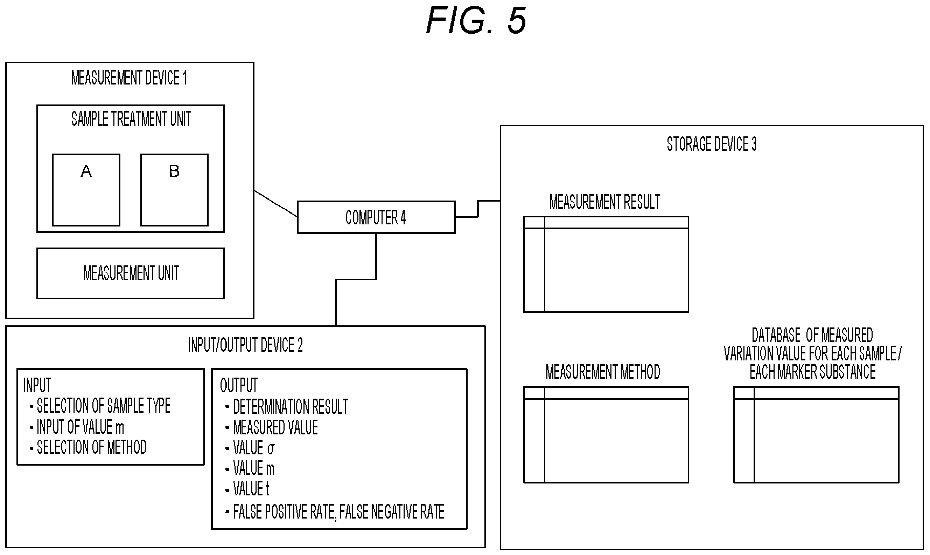

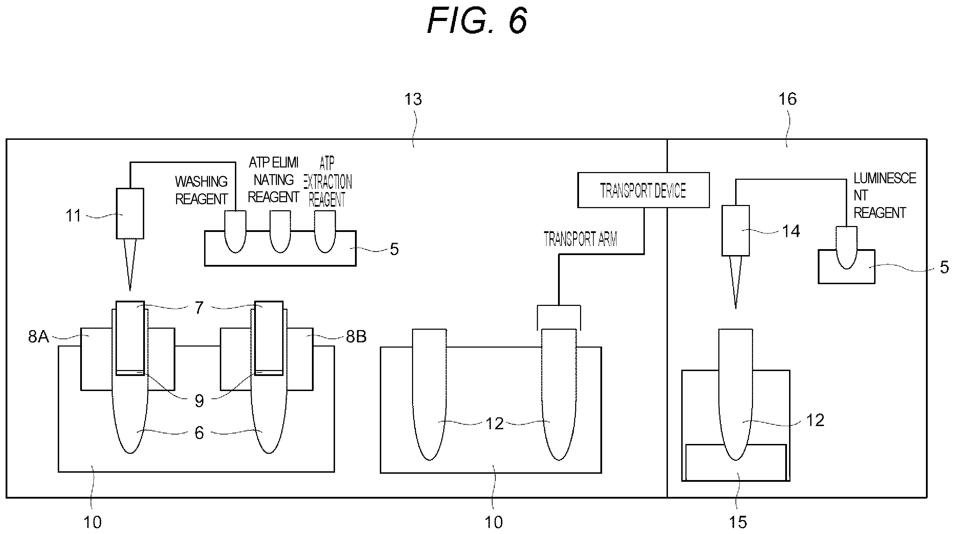

FIG. 5 shows a configuration example of the microorganism detection apparatus according to the present invention. A measurement device 1, an input/output device 2, and a storage device 3 are connected to a computer 4. Further, FIG. 6 shows a configuration example of the measurement device (a sample treatment unit 13 and a measurement unit 16). A case where the presence or absence of bacteria is determined by measuring ATP in a sample will be described.

A user may select the type of the sample, the coefficient m for calculating the threshold, and the measurement method in the input/output device 2.

Further, the user may place a washing reagent, an ATP eliminating reagent, an ATP extraction reagent, and a luminescent reagent in reagent holders 5 in the measurement device.

The user may also place, for two sets of partial samples A and B, waste liquid containers 6 and sample containers 7 with filtration filters 9 in separate temperature control units 8 (8A and 8B), respectively. The temperature control units 8A and 8B are configured to control the temperature of the filters 9 and surroundings thereof, and the temperatures may be set independently. The filters 9 to be used may be sterile filters capable of capturing bacteria and having a pore size of 0.45 µm or less.

For example, the user may divide the test sample into two equal portions and may add the divided portions to the sample containers 7, respectively.

The sample containers 7 and the waste liquid containers 6 may be set in a centrifuge rotor 10, filtration may be performed by centrifugation, bacteria may be captured and concentrated on filters, and solvents may be stored in the waste liquid containers 6. Removal of the solvents may result in removing a substance that inhibits the functions of the reagents to be added later and a substance that inhibits the growth of bacteria, and thus it may be possible to perform highly sensitive measurement.

Here, although the sample containers 7 and the waste liquid containers 6 are placed in the centrifuge rotor 10, tube holders may be provided separately, and the sample containers 7 and the waste liquid containers 6 may be transported to the centrifuge rotor at the time of performing centrifugal filtration. Instead of centrifugal filtration, filtration can be performed by other means such as aspiration filtration or pressure filtration.

Next, a washing reagent for filter washing may be added using a dispensing device 11 and then removed by centrifugal filtration. As the washing reagent, a solution that does not damage bacteria and can wash the sample matrix, such as a buffer solution, an enrichment medium, a surfactant solution, or an ATP eliminating reagent, may be selected.

Next, the filter may be heated by the temperature control unit 8A, and thus the bacteria on the filter may be sterilized. The treatment may be performed at a temperature of about 60 to 80°C for about 1 minute to 10 minutes.

Next, the dispensing device 11 may be used to add the ATP eliminating reagent to both the sample containers 7 of A and B, and the extracellular free ATP remaining on the filters may be eliminated. At this time, under appropriate conditions, viable bacteria on the filter 9 of B may grow, and bacteria-derived ATP may increase. When the elimination of extracellular ATP may be performed along with the enrichment of bacteria as described above, a substance or culture medium serving as a nutrient source for promoting the growth of bacteria may be added to the ATP eliminating reagent. Each of the temperature control units 8A and 8B may be set to a temperature around 35°C suitable for the ATP elimination and the enrichment of bacteria, and the reaction may be performed for a certain period of time. The conditions (temperature and time) of this reaction can be determined as the enrichment time required for detecting a target number of standard strains by testing the standard strains in advance. After the reaction, the ATP eliminating reagent may be removed by centrifugal filtration.

Next, the sample containers 7 may be moved to measurement sample collection containers 12. An ATP extraction reagent may be added thereto to extract intracellular ATP. Thereafter, the extract may be recovered in a recovery container by centrifugation of the centrifuge rotor 10.

After the collection, the measurement sample collection container 12 may be transported from the sample treatment unit 13 to the luminescence measurement unit 16, and the sample container 7 may be removed. A luminescent reagent may be added to the extract by a dispensing device 14, and the produced luminescence may be measured by a photodetector 15.

Here, the sample collection container 12 may be transported from the centrifuge rotor 10 to the photodetector 15, but the photodetector 15 may be positioned at the lower part of the centrifuge rotor 10 and luminescence measurement may be performed on the spot. In this case, the sample treatment unit and the measurement unit may be integrated.

The computer 4 may read out the measured variation value σ corresponding to the type of sample selected by the user and the marker substance (ATP in this example) to be measured from the database of the storage device 3, and may calculate the threshold t = a + m · σ based on the value m set by the user and the measured value a of the sample A measured by the photodetector 15. The measured value b of the sample B may be compared with the threshold t. The sample may be determined to be positive for bacteria when the value b is larger than the threshold t, and the sample may be determined to be negative for bacteria when the value b is smaller than the threshold t. The sample name, the measured values (a, b), σ, m, and t, and the determination result may be stored in the storage device, and the determination result may be output to the input/output device (monitor) 2. The false positive rate and/or false negative rate may be output as necessary.

With the above device configuration, an optimal detection threshold can be set for each test sample, and bacteria can be detected more rapidly.

### Example 3

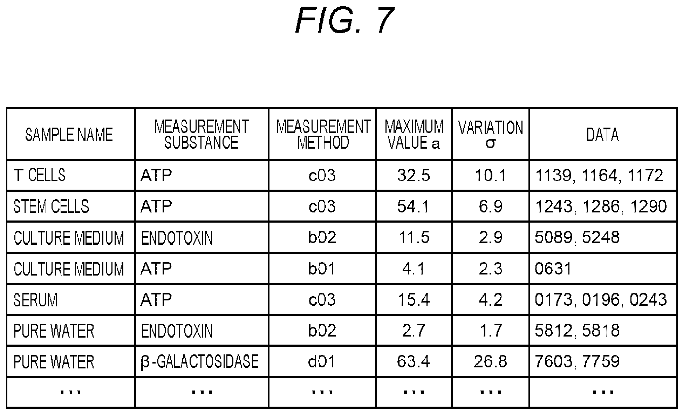

FIG. 7 shows an example of a database used in the apparatus according to the present invention.

In the database, the type of the sample, the measurement substance (marker substance), the measurement method, the maximum value of the measured value, the variation σ of the measured value, and data used to calculate these values may be recorded in association with each other. The measurement method may be a method of pretreating and measuring a sample, and may include information such as the reagent type, addition amount, temperature conditions, reaction time, filtration conditions, and setting of the photodetector.

The measurement substance may not be limited to one type, and may include a plurality of types of substances. The measurement substance can be appropriately selected according to the type of bacteria to be detected and the type of the test sample. Examples of the substance that can be quantified by luminescence measurement may include ATP, endotoxin, and β-galactosidase. A reagent capable of quantifying the amount and/or activity of these substances by luminescence measurement may be used, as a result of which one device allows bacteria to be detected by various substances. Further, it may also be possible to quantify substances derived from bacteria using fluorescence measurement or quantify genes derived from bacteria by quantitative PCR.

In the measurement of the test sample, the user may specify a sample and a measurement substance. The computer may calculate the threshold using the corresponding σ value from the database. The maximum value a may be stored in the database, and thus, when the measured value a of the sample A exceeds the maximum value, a warning can be displayed on the output screen as a suspected error (or an audio warning is issued) in the treatment of the sample.

### Example 4

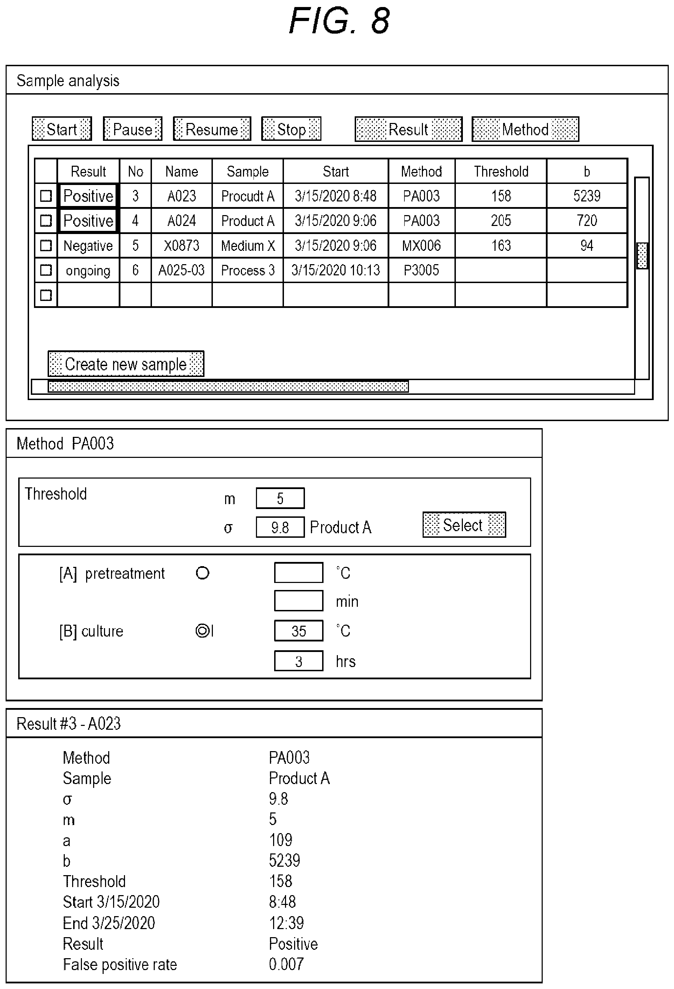

FIG. 8 shows an example of an input/output screen used in the apparatus according to the present invention.

On the Sample analysis screen, a sample name (Name), a sample type (Sample), and a measurement method (Method) can be selected, and an instruction to perform measurement can be issued. After the measurement, the automatically calculated detection threshold (Threshold), the measured value (b) of the sample B, and the determination result (Result) may be displayed.

On the measurement method (Method) screen, the value m necessary for calculating the threshold (Threshold) may be set. Further, the value of σ to be used may be read from the database. It may also be possible to set the temperature of the sterilization treatment (pretreatment) and the temperature of the enrichment culture (culture).

On the measurement result (Result) screen, the measurement method (Method), the sample type (Sample), the value σ, the value m, the measurement results (a and b), the calculated threshold (Threshold), the measurement execution date and time (Start and End), the determination result (Result), the false positive rate determination (False positive rate), and the like may be displayed. This information may be stored in the storage device.

### Reference Signs List

- 1: measurement device
- 2: input/output device
- 3: storage device
- 4: computer
- 5: reagent holder
- 6: waste liquid container
- 7: sample container
- 8: temperature control unit (8A, 8B)
- 9: filter
- 10: centrifuge rotor
- 11: dispensing device
- 12: measurement sample collection container
- 13: sample treatment unit
- 14: dispensing device
- 15: photodetector
- 16: measurement unit

## Claims

1. A method for detecting a microorganism in a sample, comprising:
preparing a test sample;
preparing, as a standard sample, a sample of the same type as the test sample;
measuring a marker substance in the standard sample a plurality of times, the marker substance being present in the microorganism or being secreted by the microorganism;
calculating a variation σ of a plurality of resulting measured values;
dividing the test sample to prepare at least two partial samples;
performing different treatments on a first partial sample and a second partial sample;
measuring the marker substance in the first partial sample to obtain a first measured value;
measuring the marker substance in the second partial sample to obtain a second measured value;
calculating an average value a of the first measured value;
calculating a threshold from the first measured value, wherein the threshold t is calculated by t = a + m · σ (where m is any coefficient); and
comparing the second measured value with the threshold to determine whether or not the microorganism is contained in the sample.

2. The method according to claim 1, wherein the sample of the same type as the test sample is a bacteria-free sample.

3. The method according to claim 1, wherein the treatment to be performed on the first partial sample comprises a treatment of inhibiting measurement of the marker substance.

4. The method according to claim 1, wherein the treatment to be performed on the second partial sample comprises a treatment for increasing an amount of the marker substance.

5. The method according to claim 1, further comprising determining a time of the treatment for increasing the amount of the marker substance of the microorganism, based on a known amount of the marker substance of the microorganism, a known growth rate of the microorganism, and the variation σ.

6. The method according to claim 1, wherein the sample is selected from the group consisting of a cell sample, a pharmaceutical or cosmetic sample, a culture medium sample, a body fluid sample, an environmental sample, a food sample, and a water sample.

7. The method according to claim 1, wherein the marker substance comprises at least one selected from the group consisting of ATP, endotoxin, nicotinamide adenine dinucleotide, gene, and β-galactosidase.

8. A microorganism detection apparatus, comprising:
a measurement unit that is configured to measure a marker substance in at least two partial samples prepared by dividing a test sample, the marker substance being present in the microorganism or being secreted by the microorganism;
a sample treatment unit having at least two sample containers, wherein, in the sample containers of the sample treatment unit, different treatments are performed on the at least two partial samples,
a storage unit that is configured to store a value σ of variation in marker substance measured value for a sample of the same type as the test sample, the value σ being obtained in advance; and
a control/analysis unit,
wherein the control/analysis unit is configured to calculate a detection threshold based on a measured value a of one of the partial samples measured by the measurement unit and the variation value σ from the storage unit, wherein the threshold t is calculated by t = a + m · σ (where m is any coefficient), and
to compare a measured value of the other partial sample measured by the measurement unit with the threshold, and determine whether or not the microorganism is contained in the test sample.

9. The apparatus according to claim 8, wherein the measurement unit comprises a photodetector.

10. The apparatus according to claim 8, further comprising an input unit configured to input, to the control/analysis unit, at least one type of information selected from the group consisting of a type of the test sample, a type of the marker substance, and a method for measuring the marker substance.

11. The apparatus according to claim 8, further comprising an output unit configured to output a determination result in the control/analysis unit.

## Patentansprüche

1. Verfahren zum Detektieren eines Mikroorganismus in einer Probe, umfassend:
Herstellen einer Testprobe;
Herstellen einer Probe desselben Typs wie die Testprobe als Standardprobe;
Messen einer Markersubstanz in der Standardprobe eine Vielzahl von Malen, wobei die Markersubstanz in dem Mikroorganismus vorhanden oder durch den Mikroorganismus sezerniert wird;
Berechnen einer Variation σ einer Vielzahl von resultierenden gemessenen Werten;
Teilen der Testprobe, um zumindest zwei Teilproben herzustellen;
Durchführen verschiedener Behandlungen auf einer ersten Teilprobe und einer zweiten Teilprobe;
Messen der Markersubstanz in der ersten Teilprobe, um einen ersten gemessenen Wert zu erhalten,
Messen der Markersubstanz in der zweiten Teilprobe, um einen zweiten gemessenen Wert zu erhalten;
Berechnen eines Mittelwerts a des ersten gemessenen Werts;
Berechnen eines Schwellenwerts ausgehend von dem ersten gemessenen Wert, wobei der Schwellenwert t durch t = a + m · σ (worin m ein beliebiger Koeffizient ist) berechnet wird; und
Vergleichen des zweiten gemessenen Werts mit dem Schwellenwert, um zu bestimmen, ob der Mikroorganismus in der Probe enthalten ist oder nicht.

2. Verfahren nach Anspruch 1, wobei die Probe desselben Typs wie die Testprobe eine bakterienfreie Probe ist.

3. Verfahren nach Anspruch 1, wobei die auf der ersten Teilprobe durchzuführende Behandlung eine Behandlung des Inhibierens der Messung der Markersubstanz umfasst.

4. Verfahren nach Anspruch 1, wobei die auf der zweiten Teilprobe durchzuführende Behandlung eine Behandlung zum Erhöhen der Menge der Markersubstanz umfasst.

5. Verfahren nach Anspruch 1, das weiters das Bestimmen eines Zeitpunkts der Behandlung zum Erhöhen der Menge der Markersubstanz des Mikroorganismus basierend auf einer bekannten Menge der Markersubstanz des Mikroorganismus, einer bekannten Wachstumsrate des Mikroorganismus und der Variation σ umfasst.

6. Verfahren nach Anspruch 1, wobei die Probe aus der aus einer Zellprobe, einer pharmazeutischen oder kosmetischen Probe, einer Kulturmediumprobe, einer Körperflüssigkeitsprobe, einer Umweltprobe, einer Lebensmittelprobe und einer Wasserprobe bestehenden Gruppe ausgewählt ist.

7. Verfahren nach Anspruch 1, wobei die Markersubstanz zumindest eines umfasst, das aus der aus ATP, Endotoxin, Nicotinamidadenindinukleotid, Gen und β-Galactosidase bestehenden Gruppe ausgewählt ist.

8. Mikroorganismus-Detektionsvorrichtung, umfassend:
eine Messeinheit, die ausgelegt ist, um eine Markersubstanz in zumindest zwei Teilproben zu messen, die durch Teilen einer Testprobe hergestellt werden, wobei die Markersubstanz in dem Mikroorganismus vorhanden ist oder durch den Mikroorganismus sezerniert wird;
eine Probenbehandlungseinheit, die zumindest zwei Probenbehälter aufweist,
wobei in den Probenbehältern der Probenbehandlungseinheit verschiedene Behandlungen auf den zumindest zwei Teilproben durchführt werden,
eine Speichereinheit, die ausgelegt ist, um einen Wert σ einer Variation eines gemessenen Werts der Markersubstanz für eine Probe desselben Typs wie die Testprobe zu speichern, wobei der Wert σ im Vorhinein erhalten wird; und
eine Steuer-/Analyseeineheit,
wobei die Steuer-/Analyseeinheit ausgelegt ist, um einen Detektionsschwellenwert basierend auf einem gemessenen Wert a einer der Teilproben, der durch die Messeinheit gemessen wird, und dem Variationswert σ aus der Speichereinheit zu berechnen, wobei der Schwellenwert t durch t = a + m · σ (worin m ein beliebiger Koeffizient ist) berechnet wird, und
um einen gemessenen Wert der anderen Teilprobe, der durch die Messeinheit gemessen wird, mit dem Schwellenwert zu vergleichen und um zu bestimmen, ob der Mikroorganismus in der Testprobe vorhanden ist oder nicht.

9. Vorrichtung nach Anspruch 8, wobei die Messeinheit einen Photodetektor umfasst.

10. Vorrichtung nach Anspruch 8, die weiters eine Eingabeeinheit umfasst, die ausgelegt ist, um zumindest einen Typ von Informationen, ausgewählt aus der aus einem Typ der Testprobe, einem Typ der Markersubstanz und einem Verfahren zum Messen der Markersubstanz bestehenden Gruppe, in die Steuer-/Analyseeinheit einzugeben.

11. Vorrichtung nach Anspruch 8, die weiters eine Ausgabeeinheit umfasst, die ausgelegt ist, um ein Bestimmungsergebnis in der Steuer-/Analyseeinheit auszugeben.

## Revendications

1. Procédé de détection d'un micro-organisme dans un échantillon, comprenant les étapes consistant à :
préparer un échantillon test ;
préparer, en tant qu'échantillon étalon, un échantillon du même type que l'échantillon test ;
mesurer une substance de marquage dans l'échantillon étalon une pluralité de fois, la substance de marquage étant présente dans le micro-organisme ou étant sécrétée par le micro-organisme ;
calculer une variation σ d'une pluralité de valeurs mesurées résultantes ;
diviser l'échantillon test afin de préparer au moins deux échantillons partiels ;
effectuer des traitements différents sur un premier échantillon partiel et un second échantillon partiel ;
mesurer la substance de marquage dans le premier échantillon partiel afin d'obtenir une première valeur mesurée ;
mesurer la substance de marquage dans le second échantillon partiel afin d'obtenir une seconde valeur mesurée ;
calculer une valeur moyenne a de la première valeur mesurée ;
calculer un seuil à partir de la première valeur mesurée, dans lequel le seuil t est calculé par t = a + m · σ (où m est un coefficient quelconque) ; et
comparer la seconde valeur mesurée au seuil afin de déterminer si le micro-organisme est contenu ou non dans l'échantillon.

2. Procédé selon la revendication 1, dans lequel l'échantillon du même type que l'échantillon test est un échantillon exempt de bactéries.

3. Procédé selon la revendication 1, dans lequel le traitement à effectuer sur le premier échantillon partiel comprend un traitement d'inhibition de la mesure de la substance de marquage.

4. Procédé selon la revendication 1, dans lequel le traitement à effectuer sur le second échantillon partiel comprend un traitement pour augmenter une quantité de la substance de marquage.

5. Procédé selon la revendication 1, comprenant en outre la détermination d'un temps du traitement pour augmenter la quantité de la substance de marquage du micro-organisme, sur la base d'une quantité connue de la substance de marquage du micro-organisme, d'un taux de croissance connu du micro-organisme, et de la variation σ.

6. Procédé selon la revendication 1, dans lequel l'échantillon est choisi dans le groupe constitué d'un échantillon cellulaire, d'un échantillon pharmaceutique ou cosmétique, d'un échantillon de milieu de culture, d'un échantillon de fluide corporel, d'un échantillon environnemental, d'un échantillon alimentaire et d'un échantillon d'eau.

7. Procédé selon la revendication 1, dans lequel la substance de marquage comprend au moins un élément choisi dans le groupe constitué d'ATP, endotoxine, nicotinamide adénine dinucléotide, gène et β-galactosidase.

8. Appareil de détection de micro-organisme, comprenant :
une unité de mesure qui est configurée pour mesurer une substance de marquage dans au moins deux échantillons partiels préparés en divisant un échantillon test, la substance de marquage étant présente dans le micro-organisme ou étant sécrétée par le micro-organisme ;
une unité de traitement d'échantillons comportant au moins deux récipients d'échantillon,
dans lequel, dans les récipients d'échantillon de l'unité de traitement d'échantillons, des traitements différents sont effectués sur les au moins deux échantillons partiels ;
une unité de stockage qui est configurée pour stocker une valeur σ de variation de valeur mesurée de substance de marquage pour un échantillon du même type que l'échantillon test, la valeur σ étant obtenue à l'avance ; et
une unité de commande/analyse,
dans lequel l'unité de commande/analyse est configurée pour calculer un seuil de détection sur la base d'une valeur mesurée a de l'un des échantillons partiels mesurée par l'unité de mesure et de la valeur de variation σ provenant de l'unité de stockage, dans lequel le seuil t est calculé par t = a + m · σ (où m est un coefficient quelconque), et
pour comparer une valeur mesurée de l'autre échantillon partiel mesurée par l'unité de mesure au seuil, et déterminer si le micro-organisme est contenu ou non dans l'échantillon test.

9. Appareil selon la revendication 8, dans lequel l'unité de mesure comprend un photodétecteur.

10. Appareil selon la revendication 8, comprenant en outre une unité d'entrée configurée pour entrer, dans l'unité de commande/analyse, au moins un type d'informations choisi dans le groupe constitué d'un type de l'échantillon test, d'un type de la substance de marquage et d'un procédé de mesure de la substance de marquage.

11. Appareil selon la revendication 8, comprenant en outre une unité de sortie configurée pour délivrer en sortie un résultat de détermination dans l'unité de commande/analyse.
